(19)

**Europäisches Patentamt**
**European Patent Office**
**Office européen des brevets**

(11) **EP 3 422 261 A1**

(12) **EUROPEAN PATENT APPLICATION**
published in accordance with Art. 153(4) EPC

(43) Date of publication:
**02.01.2019 Bulletin 2019/01**

(21) Application number: **17899753.2**

(22) Date of filing: **21.06.2017**

(51) Int Cl.:
**G06N 3/12** $^{(2006.01)}$

(86) International application number:
**PCT/CN2017/089285**

(87) International publication number:
**WO 2018/161468 (13.09.2018 Gazette 2018/37)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **10.03.2017 CN 201710140258**
**12.06.2017 CN 201710437829**

(71) Applicants:
• **Dongguan University of Technology
Dongguan, Guangdong 523808 (CN)**
• **Li, Yun
Dongguan, Guangdong 523808 (CN)**

• **Li, Lin
Dongguan, Guangdong 523808 (CN)**

(72) Inventors:
• **LI, Yun
Dongguan
Guangdong 523808 (CN)**
• **LI, Lin
Dongguan
Guangdong 523808 (CN)**

(74) Representative: **2K Patentanwälte Blasberg
Kewitz & Reichel
Partnerschaft mbB
Schumannstrasse 27
60325 Frankfurt am Main (DE)**

(54) **GLOBAL OPTIMIZATION, SEARCHING AND MACHINE LEARNING METHOD BASED ON LAMARCK ACQUIRED GENETIC PRINCIPLE**

(57) The invention discloses a global optimization, search and machine learning method based on the Lamarckian principle of inheritance of acquired characteristics, comprising step 1: constructing an objective function $f(\mathbf{P})$ according to the problem being solved, where $\mathbf{P}$ represents a set of candidate solutions to the problem; step 2: encoding $\mathbf{P}$ into a genetic algorithm (GA) chromosome, inputting or automatically calculating algorithmic parameters of the GA, and initializing the algorithm and the population of candidate solution generation $\mathbf{G}_0=\{\mathbf{P}_0^1, \mathbf{P}_0^2, ..., \mathbf{P}_0^S\}$, where $S$ is the size of the population $\mathbf{G}$ and 0 stands for the initial generation; step 3: at generation $k$, optimizing the prevailing population of the candidate solutions $\mathbf{G}_k=\{\mathbf{P}_k^1, \mathbf{P}_k^2, ..., \mathbf{P}_k^S\}$ iteratively using a Lamarckian *"Heredity Operator"* and a *"Use-and-Disuse Operator"* based on the values of $f(\mathbf{G}_k)$; and step 4: outputting the final set of optimal solutions to the problem.

FIG. 1

**Description**

BACKGROUND OF THE INVENTION

1. Field of the Invention

The present invention is related to the field of computing technology, including artificial intelligence, especially a global optimization, search and machine learning method based on the genetic or evolutionary algorithm.

2. Description of Related Art

To solve practical problems, such as a global optimization, search, or machine learning problem, a generic genetic algorithm (GA) uses the "Survival of the fittest" law of Darwinism evolution, which repeatedly applies three operators: a selection operator, a crossover operator and a mutation operator. The GA has low search speed and low search accuracy. Further, the GA performs poorly in local search, thereby leading to low search efficiency in the evolutionary computing process. Also, the GA is prone to causing premature convergence in practical applications. Obtaining a method to keep good individuals and to maintain population diversity is always the more difficult technical problem in the genetic algorithm, which limits its applicability to more real-world problems including global optimization, search and machine learning.

SUMMARY OF THE INVENTION

To overcome the abovementioned technical problems of a generic genetic algorithm, which is based on the "Survival of the Fittest" law of Darwinian evolution, this invention combines this natural law of Darwinism evolution with the Lamarckian principle of inheritance of acquired characteristics and the modern "Epigenetics" to construct a "Heredity Optimization" method and a "Heredity Algorithm" (HA). First, according to the natural law - the "Inheritance of acquired characteristics" of Lamarckian evolution, a *"Heredity Operator"* and its *"Overwriting Operation"* method are invented to substitute two operators (namely, "Selection Operator" and "Crossover Operator") of a current genetic algorithm directly, thereby simplifying the structure of the genetic algorithm while overcoming multiple technological defects of current genetic algorithms and improving global optimality and sustainability of later evolution of the genetic algorithm. Second, according to the natural law - the "Use and Disuse" of Lamarckian evolution, a *"Use-and-Disuse Operator"* is invented to substitute an inborn mutation operator in the generic genetic algorithm; therefore, directed mutation operations are implemented within the generation, which makes mutation operations generate new technical effects, improve the performance of the genetic algorithm and solve problems of global optimization, search and machine learning even more and better.

A global optimization, search and machine learning method based on the Lamarckian principle of inheritance of acquired characteristics, comprising:

Step 1, constructing an objective function $f(\mathbf{P})$ according to the problem being solved for optimization, search or machine learning, where $\mathbf{P}$ represents a population set of $S$ candidate solutions to the problem;

Step 2, encoding the population set into a genetic algorithm (GA) chromosome set comprising L genes corresponding to the number of variables of the problem and its optimization, search or machine learning needs, where each chromosome represents an individual of the population. Then, inputting or automatically calculating algorithmic parameters of the GA, and initializing the algorithm and the population at the $0th$ generation;

Step 3, supposing that the prevailing population set of candidate solutions at the $k$th generation is $\mathbf{G}_k=\{\mathbf{P}_k^1, \mathbf{P}_k^2, ..., \mathbf{P}_k^S\}$, where $\mathbf{P}_k^i$ indicates the $i$th individual chromosome in the $k$th generation $\mathbf{G}_k$, and Sis the population size, obtaining through iteration the population for the $(k+1)th$ generation $\mathbf{G}_{k+1}=\{\mathbf{P}_{k+1}^1, \mathbf{P}_{k+1}^2, ..., \mathbf{P}_{k+1}^S\}$, where the optimization process comprises the following steps:

(1) individual evaluation: calculating the corresponding value of objective function $f(\mathbf{P})$ for every $\mathbf{P}_k^i$ after decoding its chromosome;

(2) heredity operation: applying Lamarckian *"Heredity Operator"* to generate a temporary population $\mathbf{G}_{k+1}$, specifically including the sub-steps of:

(2a) selecting randomly two chromosomes from the candidate population $\mathbf{G}_k$ according to a crossover inheritance probability $p_c$ initialized in step 2, comparing objective function values $f_m$ and $f_n$ of the two chromosomes, and calculating a genetic heredity proportion $p_t$:

$$p_t = f_m/(f_m + f_n), f_m > f_n;$$

(2b) calculating the number of genes to be passed onto the next generation:

$$n_t = L \cdot p_t;$$

(2c) while retaining chromosome $m$, overwriting $n_t$ genes at random positions onto chromosome n at corresponding positions to form a new chromosome;

(2d) repeating steps (2a)-(2c) for $p_cS$ times to generate the temporary population $\mathbf{G}'_{k+1}$;

(3) exercising a directed mutation operation for the temporary population $\mathbf{G}'_{k+1}$ with Lamarckian *"Use-and-Disuse Operator"* and hence forming the $(k+1)th$ generation of candidate solutions $\mathbf{G}_{k+1}$;

(4) iterating steps (1)-(3) until the termination condition pre-specified at initialization is met;

(5) evaluating whether the best solutions in the terminating generation meet the requirements of this optimization, search or machine learning problem; if not, revising the algorithmic parameters and repeating steps (1)-(4) again until the final requirements are met;

Step 4, outputting the final solutions set and terminating the algorithm.

In the step 2, the initializing method comprises following steps:

(1) First, confirming the algorithmic parameters according to an operation mode of a genetic algorithm, including the candidate solution population size $S$, the variable dimensionality $d$, the ranges of possible values of the variables, the number of genes in a chromosome $L$, the crossover inheritance probability $p_c$, and internal parameters in the mutation operator;

(2) Then, encoding the solution set of the problem to form individual genes, chromosomes and the candidate solution population, with length L of the chromosome;

(3) Last, initializing the algorithm according to the ranges of the variable values of the problem and generating one group of initial candidate solutions randomly, wherein when $k=0$, a gene group of each chromosome $\mathbf{P}_0^i$ is $\{x_0^1(i), x_0^2(i), ..., x_0^s(i)\}$, namely $\mathbf{P}_0^i = \{x_0^j(i), j=1,...,d\}$; the initial population is $\mathbf{G}_0 = \{\mathbf{P}_0^i, i=1,2,...,S\}$; $S$ is the population size, and $d$ is the variable dimensionality.

In the step 2 (2), the solution set of the problem is encoded with methods: using binary or decimal encoding if $d$ is less than or equal to 2, or using real number encoding method if $d$ is greater than 2.

The objective function value is a fitness function value for a maximization problem or a cost function value for a minimization problem.

In the step 3(1), the individual evaluation is implemented with multiple processors or multiple computers.

In the step 3(2), $n_t$ genes for overwriting are randomly selected during the overwriting operation of the Lamarckian heredity operator.

In the step 3(3), the mutation operation is carried out by non-directed mutation method in a generic genetic algorithm, including the uniform mutation method with mutation probability $p_m$, which don't utilize the *"Use-αnd disuse operator"*.

In the step 3(3), the mutation operation can also be carried out by the "Use and disuse operator" based on the natural law - the *"Use-and-disuse"* of Lamarckian evolution, namely the directed mutation method.

In the step 3(3), gradient optimization methods can be applied to the "Use-and-disuse operator" if the gradient information is acquired; and mutation directions and step lengths can be confirmed according to the sign and size of the gradient on the premise that the gradient information is acquired.

In the step 3(3), non-gradient optimization methods can be applied to the "Use-and-disuse operator", which including the Hill-climbing algorithm, the Annealing algorithm, the Simplex method, the Pattern Search or the Powell's method and so on.

In the step 3(2) and step 3(3), an elite chromosome in each generation shall not be changed by the Lamarckian heredity operator and the mutation operator, namely retain the elite without overwriting the best individual (also called the elite individual, or the elite chromosome) of the mutation population so far occurring due to participation in evolution; however, the best individual shall be copied directly in the next generation. The methods of confirming the elite are the methods of the generic algorithm.

In the step 3(5), the algorithm also includes steps: elevating an optimal solution whether meets the requirements of this optimal computation; if so, the operation ends; otherwise, crossover inheritance probability $p_c$ in algorithmic parameters or internal parameters in the mutation operator can be modified, and repeating the step 3 to acquire the optimal solution set of the final generation of the problem.

The crossover inheritance probability $p_c$ in algorithmic parameters and internal parameters in the mutation operator, which are confirmed in the step 2, can be adjusted automatically according to the evolutional status during the step 3.

In step 2, a gene code on the chromosome can indicate the solution structure of the problem or numerical parameters of the structure.

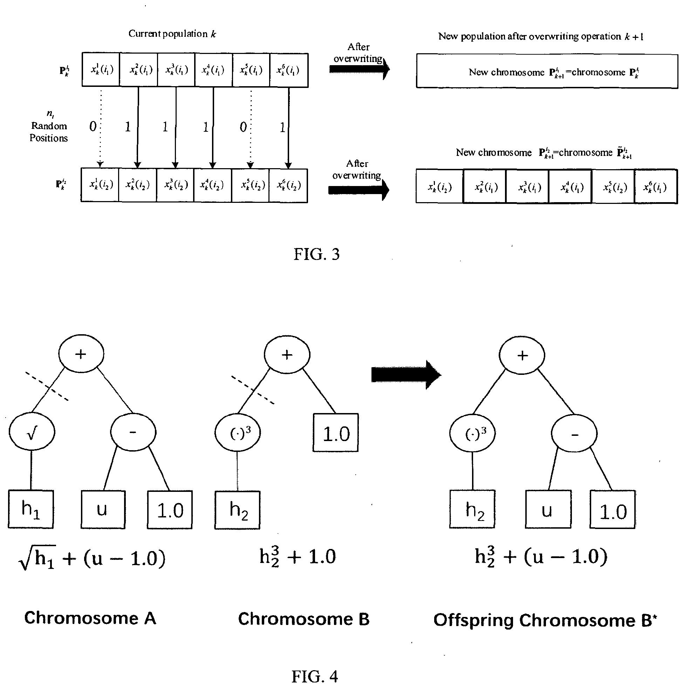

Generally, the problem is composed of the problem structure, numerical parameters of each structure, and combination methods between structures (some of them are represented by arithmetic operators or logical operators). In the generic genetic algorithm, usually a gene code on the chromosome is not an operator; in "Genetic Programming", a gene code on the chromosome can be an operator. In the step 2 of this invention, the gene code of the problem's solution set can be an operator. Therefore, the Lamarckian Heredity Algorithm of this invention is expanded to "Heredity Programming", and the problem's structure can be optimized freely and substantially. "Heredity Operator" in the Heredity Programming is similar to the crossover operation of Genetic Programming, as shown in Fig. 4. However, a tree-like "Pruning" of chromosome is carried out through the overwriting operation in the Heredity Algorithm. Similarly, a certain small branch can be substituted by a new branch with the mutation operation and the "Use and Disuse" operator. Therefore, the method of this invention for implementing the heredity operator and the "Use and Disuse" operator in the Heredity Programming has not been changed. But the speed and accuracy of Genetic Programming are improved. In this way, the inheritance of acquired characteristics can improve the functions of free structure optimization, automatic programming attempt and machine learning.

This invention combines the Lamarckian principle of inheritance of acquired characteristics with modern "Epigenetics" and the natural law - "Survival of the fittest" in Darwinism to construct a heredity optimization technical solution, a heredity algorithm and a heredity programming method. It is invented to replace the selection operator and crossover operator in generic genetic algorithm by operating the heredity operator in the Lamarckian natural law of inheritance of acquired characteristics It is also invented to replace the inborn mutation operator in generic genetic algorithm by operating the "Use and disuse" operator in the Lamarckian natural law of "Use and disuse" In this way, the acquired and directed mutation of "Use and disuse" is implemented in the same generation, to provide the mutation operation with new technical effects, and to solve the problems of global optimization, search and machine learning better. This invention specifically has the following advantages:

(1) Simple optimization process, few required control parameters, low computation complexity and convenient operation;
(2) Strengthen the global optimization and the sustainability of later evolution of genetic algorithm and genetic programming;
(3) Converge the optimal solution set of problems in global optimization, searching and machine learning more quickly with higher accuracy.
(4) Have wider application prospect and field.

1) An explicit mathematical expression is not required while optimizing complicated problems; therefore, it is a powerful tool to solve this kind of problem.
2) Analysis of complicated system: the evolutionary computation shall be used to implement cluster analysis, pattern recognition, image processing, scheduling organization, etc., thereby methodizing disorganized and complicated matters.
3) Automatic control: the evolutionary computation has the intelligent functions of self-adaption, self-learning and self-organization and can adapt to environmental changes while reducing fluctuation, and ensuring controlling with high accuracy, real-time capability and speediness.
4) Automatic design of hardware: a brand-new method can be provided for the fields including complicated circuit design and self-adapting hardware.
5) Automatic program design: an automatic program design method implemented based on genetic program design is developing into the study of evolutionary software, that is, a task can be completed automatically by computer without telling it the specific method accurately.
6) Comprehensive application: this invention can play its specialty when combined with other technologies to solve problems comprehensively. For example, the genetic algorithm solves the problems of machine learning, etc. when combined with the artificial neural network.

BRIEF DESCRIPTION OF THE DRAWINGS

Fig. 1 depicts a flow chart of heredity algorithm.

Fig. 2 depicts the flow chart of rewriting operation of a "heredity operator".

Fig. 3 depicts a schematic diagram of an overwriting operation.

Fig. 4 depicts a "propagation by grafting" schematic diagram of heredity programming.

Fig. 5 depicts the algorithm fitness comparison between this invention and generic genetic algorithm in specific embodiment during optimizations of global minimum ($f_1$) and maximum ($f_2$).

Fig. 6 depicts the algorithm accuracy comparison between this invention and generic genetic algorithm in specific embodiment during optimizations of global minimum ($f_1$) and maximum ($f_2$).

Fig. 7 depicts the schematic diagram of neural network machine learning.

Fig. 8 depicts the schematic diagram of the two-variable exclusive-or problems implemented in the neural network.

Fig. 9 depicts the optimal and average function values.

Fig. 10 depicts relationship between two inputs and an output.

Fig. 11 depicts the schematic diagram of comparison curves of average root-mean-square errors between this invention and other particle filter algorithms when the number of particles is 10.

DETAILED DESCRIPTION OF THE PREFERRED EMBODIMENTS **Test**

A performance test and comparison between this invention and a generic genetic algorithm are implemented in this test through selecting standard functions of problems in global optimization, searching and machine learning. Standard functions and corresponding parameter settings are shown in Table 1:

Table 1 Standard test functions of problems in global optimization, searching and machine learning

| Test function | Dimension | Range of value | Global | Function name |
|---|---|---|---|---|
| $f_1(x) = \sum_{i=1}^{n} i x_i^4$ | 30 | $[-1.28, 1.28]^n$ | [0,1248.2] | Quartic [1] |
| $f_2(\mathbf{x}) = \sum_{i=1}^{n} f_i(x_i) = \sum_{i=1}^{n} \sin(x_i) \sin^{200}\left(\frac{i x_i^2}{\pi}\right)$ | 30 | $\mathbf{x} \in [0, \text{pi}]^n$ | [0,29.6252] | $n$-D Varying Landscape [2] |

[1]Xin Yao. Evolutionary Programming Made Faster. IEEE Trans Evolutionary Computation, 1999, 3(2):82-101.
[2]Michalewicz, Z.. Genetic Algorithms + Data Structure = Evolutionary Programs, Springer-Verlag, Berlin, 1992.

Generally, a population size can be set as 10 times of the number of variables (namely dimensionality); therefore, the population size in this embodiment is set as $S$=300. The crossover probability in the generic genetic algorithm is $p_c$=0.5. The mutation probability is $p_m$=0.2 generally. To ensure a consistent verification, the crossover inheritance probability in the invention is also 0.5, and the mutation probability is 0.2. Each test function shall be operated 30 times independently and respectively. Furthermore, the number of function evaluation is $3 \times 10^5$.

As for the two test functions, the mean values, standard differences, optimality, and accuracy results solved in searching way by the algorithm proposed in this invention and the generic genetic algorithm are shown in Table 2 and Table 3, respectively:

Table 2 Comparison of searching results in presence of the standard test function fi for problems in global optimization, searching and machine learning

| Algorithm | Theoretically optimal value | Mean value | Standard deviation | Optimality | Accuracy |
|---|---|---|---|---|---|
| Generic genetic algorithm | | 72.4849 | 16.0830 | 94.1928 | 77.9096 |
| This invention (Heredity Algorithm) | 0 | 5.9041e-08 | 3.8012e-08 | 100 | 99.9027 |

Table 3 Comparison of searching results in presence of the standard test function $f_2$ of problems for global optimization, searching and machine learning

| Algorithm | Theoretically optimal value | Mean value | Standard deviation | Optimality | Accuracy |
|---|---|---|---|---|---|
| Generic genetic algorithm | 29.6252 | 3.9849 | 0.2794 | 13.4511 | 71.2702 |
| This invention (Heredity Algorithm) | | 29.1076 | 0.0715 | 98.2528 | 95.3091 |

It is shown from statistical data in Table 2 and Table 3, that the mean value, standard deviation, optimality, and accuracy optimized by the algorithm of this invention are superior to the generic genetic algorithm. Furthermore, the accuracy of the solution is much better, which is the closest to the theoretical optimal value.

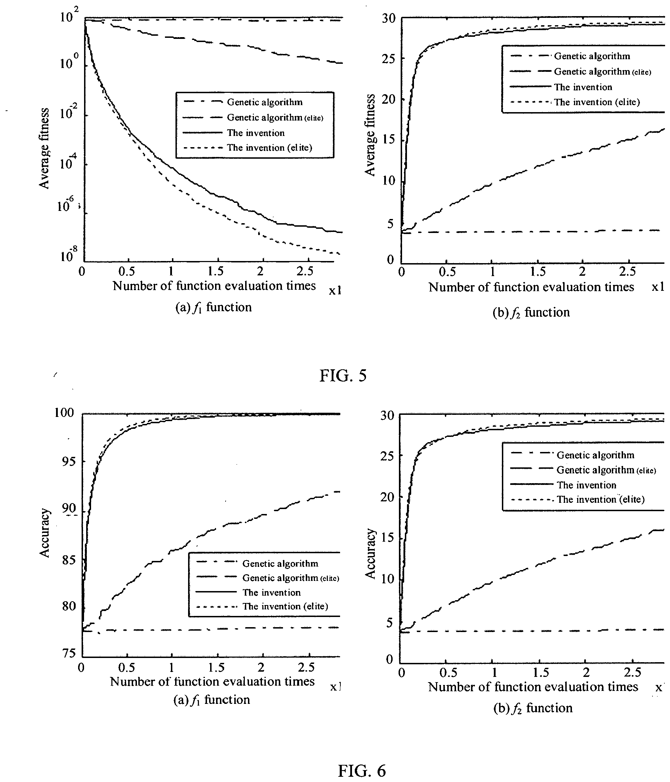

The relationship between average fitness and average searching accuracy of this invention and of the generic genetic algorithm with the variation of function evaluation times is shown in Fig. 5 and Fig. 6, respectively. From the figures, it is shown that the optimized solution obtained by the algorithm of this invention is closer to the theoretically optimal value when optimizing the test functions $f_1$ and $f_2$. Meanwhile, the searching speed of this invention is much quicker than the generic genetic algorithm.

To sum up, this invention has a higher optimization accuracy and a quicker convergence speed.

In order to facilitate public understanding and usage of this invention's technical solution, further description is provided as follows with two application examples of problems in global optimization, searching and machine learning.

**Application example 1- problem in exclusive-or machine learning based on a simple neural network**

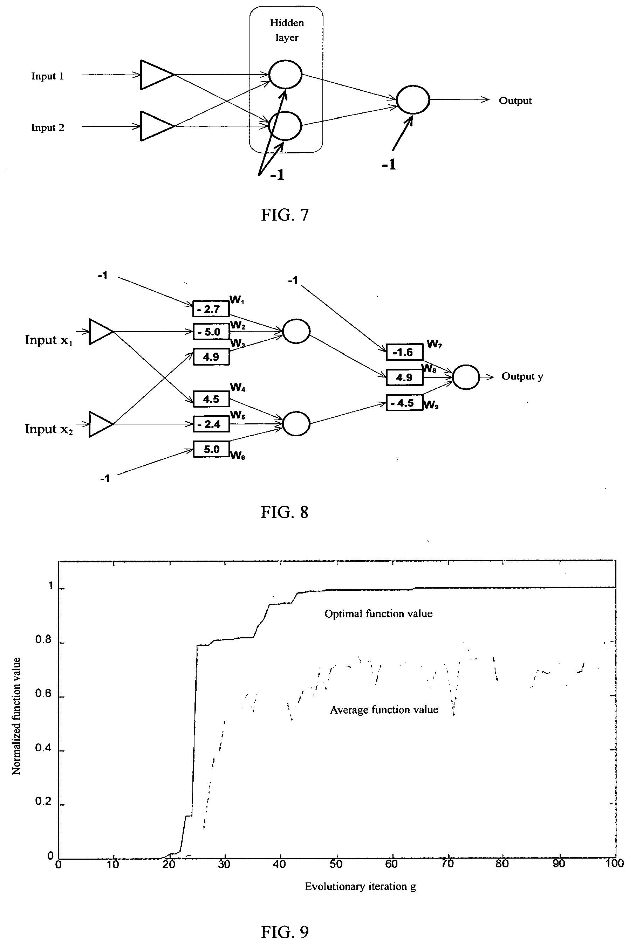

Artificial Neural Networks (ANNs for short) are also called neural networks (NNs) and are algorithm models imitating behavior characteristics of animal neural network for distributed parallel information processing and machine learning. This network realizes information processing, learning and memorization by adjusting interconnecting relationship and weight between nodes depending on interconnected nonlinear neuron nodes. A simple neural network is shown in Fig. 7, wherein a circle indicates a neuron, and an input point "-1" is called bias node. The leftmost layer of neural network is an inputting layer, and the rightmost layer is an output layer. A concealed layer is formed by all nodes in the middle, and we cannot observe their values directly in a training sample set. At the same time, in the figure, two inputting units (a bias unit is not included), two concealed units and one outputting unit are involved in the embodiment of this neural network machine learning.

In Fig. 8, one neural network with simple network is used here to implement the two-variable exclusive-or problem in order to describe the machine learning with the Heredity Algorithm; which has an important demonstration significance for finding out a practical and effective learning algorithm. If the input neurons are $\mathbf{x}_1$ and $\mathbf{x}_2$, with the output being y, four training models of exclusive-or problems are shown in Table 4.

Table 4 Training model of exclusive-or problems

| Model data $\boldsymbol{m}$ | 1 | 2 | 3 | 4 |
|---|---|---|---|---|
| Input $\boldsymbol{x_1}$ | 0 | 1 | 1 | 0 |
| Input $\boldsymbol{x_2}$ | 0 | 0 | 1 | 1 |
| Output $\boldsymbol{y}$ | 0 | 1 | 0 | 1 |

There are 9 weights in total for the connections from the neuron in the inputting layer to the neuron in the outputting layer, which are marked as $\boldsymbol{w_n}$, $n$=1, 2,...,9.

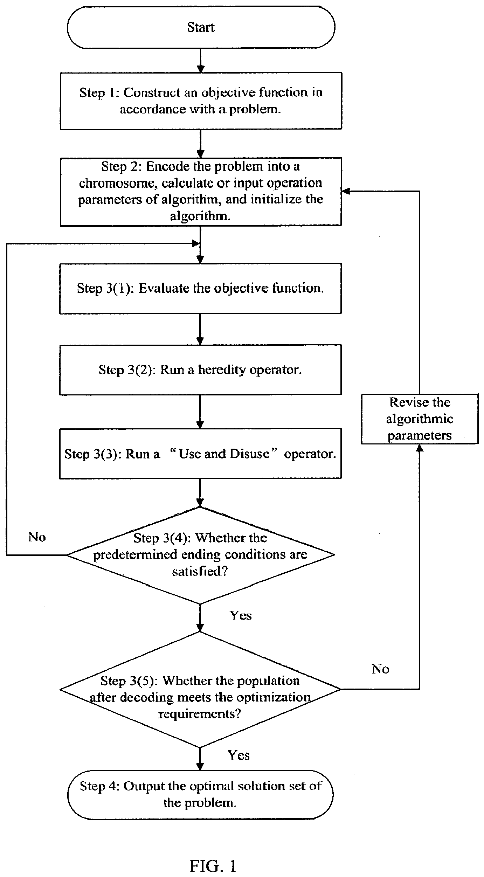

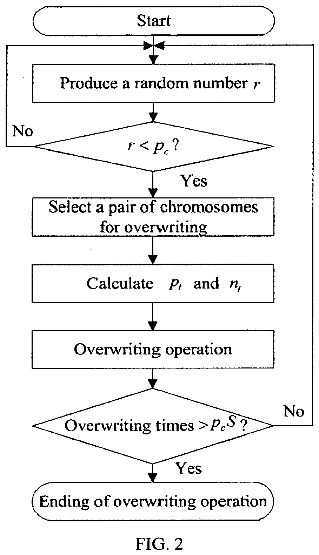

This invention's solution for global optimization, searching and machine learning is used to solve the exclusive-or problems of neural network with the weight $\boldsymbol{w_n} w_n$ of the training network. Specific execution process is shown in Fig. 1 and Fig. 2:

Step 1: construct an objective function $f(\boldsymbol{W})$ in accordance with the exclusive-or problems, wherein $\boldsymbol{W}$ is the weight vector, that is, $\boldsymbol{W}= \{\boldsymbol{w_n}, n=1,2,...,9\}$. To maintain the generality,

$$f = \left(1 + \sum_{m=1}^{4} \left| y_m - \hat{y}_m \right| \right)^{-1}$$

is used here, wherein $m$

is the training model number and, $y_{m'}$ is the output of the training model $m$. In Fig. 8, $f$ is the function of **W**, whose value indicates the solution fitness; the bigger it is, the smaller the training or learning error would be. Therefore, the target of this embodiment is to maximize $f$.

Step 2: In accordance with the optimizing demand of the problem, automatically calculate or manually input operation parameters of common genetic algorithm; code **W** into the chromosome and initialize it.

(1) First, in accordance with operation mode of common genetic algorithm, set $S$=90 (generally 10 times of parameter variables' dimension); set the variables' dimension as $d$=9, set the scope of **W** as -5.0 to 4.9; set genetic probability as $p_c$=0.6, and set mutation probability as $p_m$=0.05.

(2) Then, implement decimal encoding for the structure and its parameters which need optimization of problem to form the individual gene cluster, chromosome and candidate solution population, and to determine the gene cluster's length $L$, wherein a two-digit decimal number is used to describe the coding scheme of weights, specifically shown in Table 5. In this way, the length of gene cluster is $L$=2 × d=2 × 9=18. For example, **W**= {$w_1$, $w_2$,$w_3$,$w_4$,$w_5$,$w_6$,$w_7$,$w_8$,$w_9$}={-5,-4.9,-4.8,-4.7,0.0,4.6,4.7,4.8, 4.9}, and its coding format is **W**$_c$= {000102035096979899}

Table 5 Two-digit decimal code of weight

| Weight | - 5.0 | - 4.9 | - 4.8 | ... | 0.0 | .... | + 4.7 | + 4.8 | +4.9 |
|--------|-------|-------|-------|-----|-----|------|-------|-------|------|
| Code   | 00    | 01    | 02    | ... | 50  | .... | 97    | 98    | 99   |

(3) Finally, initialize in accordance with the value range of variables in the problem, and randomly produce one group of initial candidate solutions. When $k$=0 and the coding format of each chromosome taking $\mathbf{P}_0^i$ as weight vector is **W**$_c$ = {$x_0^1(i)$, $x_0^2(i)$,...,$x_0^L(i)$}, $\mathbf{P}_0^i$= {$x_0^j(i)$, $i$=1,...,S, $j$=1,...,L, wherein the $x_0^j(i)$ is each digit of the code, and the initial population is $\mathbf{G}_0$={$\mathbf{P}_0^i$,$i$=1,2,...,S}.

Step 3: in line with the optimization demand, it is assumed that the population of Generation-$k$ is $\mathbf{G}_k$={$\mathbf{P}_k^1$,$\mathbf{P}_k^2$,...,$\mathbf{P}_k^S$}, where $\mathbf{P}_k^i$ represents one individual (chromosome) in the population, the population of generation $k$+1$th$ $\mathbf{G}_{k+1}$={$\mathbf{P}_{k+1}^1$,$\mathbf{P}_{k+1}^2$,...,$\mathbf{P}_{k+1}^S$} can be acquired with iterative method through rewriting and mutation operation. The optimization process is as follows:

(1) Individual evaluation: calculate fitness (namely objective function value) of each individual $P_k^i$ in the population $\mathbf{G}_k$ after decoding in presence of training input matrix $\mathbf{X} = \begin{bmatrix} 0 & 1 & 1 & 0 \\ 0 & 0 & 1 & 1 \end{bmatrix}$ and anticipated output matrix $\mathbf{Y}$ = [0 1 0 1] according to Table 4; whose property belongs to the maximum problem. For example, $\mathbf{P}_k^{i_1}$ is superior to $\mathbf{P}_k^{i_2}$ when $f(\mathbf{P}_k^{i_1})$=0.4 and $f(\mathbf{P}_k^{i_2})$=0.2.

(2) As shown in Fig. 2, produce the new candidate solution population G'$_{k+1}$ by implementing the rewriting operation of Lamarckian "Heredity operator", including following steps:

(2a) Select two parent chromosomes (namely gene clusters of parent candidate solutions) randomly in accordance with the crisscross inheritance probability $p_c$, namely produce one random number $r$ with range of (0,1] randomly; select two parent chromosomes if $r < p_c$; or not select and continuously produce the random number. For example,

$$\mathbf{P}_k^{i_1} = \{x_k^1(i_1), x_k^2(i_1), \mathrm{K}\ x_k^j(i_1)\}_{j=1}^d \text{ and } \mathbf{P}_k^{i_2} = \{x_k^1(i_2), x_k^2(i_2), \mathrm{K}\ x_k^j(i_2)\}_{j=1}^d.$$

(2b) Compare fitness function values $f(P_k^{i_1})$ and $f(P_k^{i_2})$ of two chromosomes, wherein $P_k^{i_1}$ will pass on relatively more genes to the filial generations if $f(P_k^{i_1})>f(P_k^{i_2})$. The gene heredity proportion chromosomes $p_t$ is calculated by the following formula:

$$p_t = \frac{f(P_k^{i_1})}{f(P_k^{i_1}) + f(P_k^{i_2})} = \frac{0.4}{0.4 + 0.2} = \frac{2}{3}$$

Then, calculate the number $n_t$ of genes to be passed to the next generation according to the following formula:

(2c) Operate the heredity operator and implement the rewriting operation: retain the chromosomes having strong

fitness; correspondingly overwrite $n_t$ genes on the chromosome having strong fitness onto corresponding position of the chromosome with weak fitness to form new chromosomes. Correspondingly rewrite genes on the chromosome having strong fitness to corresponding position of chromosome having weak fitness. By that analogy, the basic flowchart is shown in Fig. 2, and the example of an overwriting operation is shown in Fig. 3 (not all code bits are marked in the figure). According to the last step, 18×2/3=12 coding bits in $P_k^{i2}$ are overwritten once when overwritten by the coding bit in $P_k^{i1}$.

(2d) Repeat the above-mentioned steps from (2a)-to (2c) for $p_c$ S times to produce a temporary population $\mathbf{G'}_{k+1}$ of new candidate solution after overwriting operations.

When the number of rewriting times is $N_p = p_c S$ or more (0.6×90=54, round up the product if it is not an integer), end the overwriting operation. Otherwise, continue to repeat the steps from (2a)-to (2c) when the number of overwriting times is less than 54.

(3) Implement the mutation operation for the temporary population $\mathbf{G'}_{k+1}$ by using one appropriate "Mutation operator" in order to acquire the new candidate solution population $\mathbf{G}_{k+1}$, wherein the problem of machine learning is simple; therefore, the embodiment only uses the inborn uniform mutation method in the generic genetic algorithm rather than the "Use and disuse" operations. Because the mutation probability $p_m$ =0.05, (90×18×0.05=81) code bits (round up the product if it is not the integer) in the new population $\mathbf{G}_{k+1}$ after one iteration will have the new values [0, 9].

(4) Repeat the iteration steps (1) to steps (3) until the preset termination condition is met. The termination condition applied here is simplified as 100 generations of evolution.

(5) Evaluate whether the optimal solution after decoding meets the requirements of this optimization computation; if so, finish the operation; and if not, modify operation parameters of the algorithm. For example, manually modify the crossover inheritance probability $p_c$, or the mutation probability $p_m$; increase the population size and the times of evolution iteration. Recalculate and gain the final optimized result. The requirement for optimal computation here is $f>0.95$, and the request has been met as shown in Fig. 9.

Step 4: Output the last generation's optimal solution of problem $\mathbf{W}_0$. An optimal solution set of weights is also shown in Fig. 8.

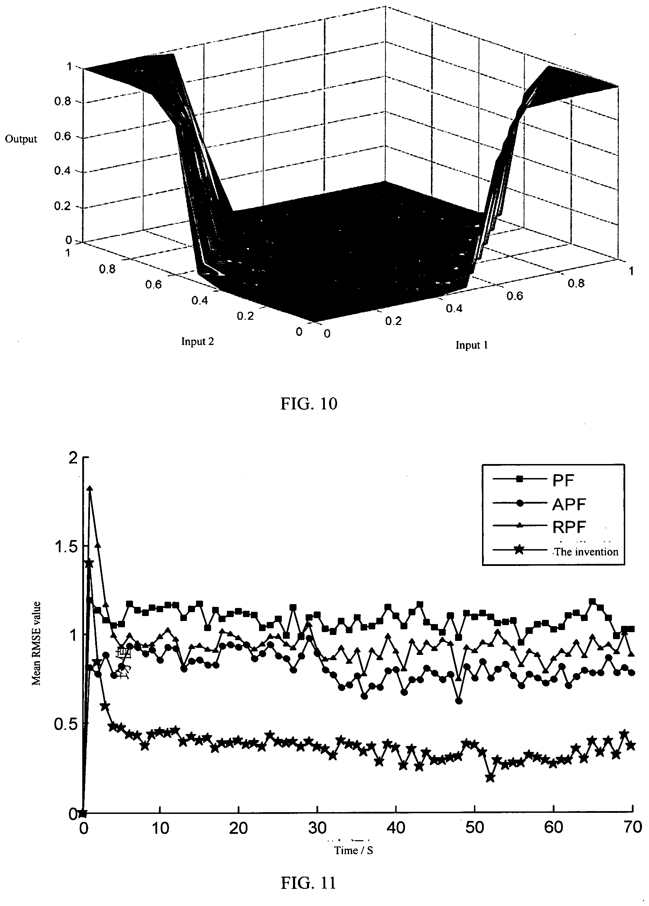

Fig. 9 and 10 respectively show the optimal and average values of the objective function after 100 times of iteration as well as the input/ output relation gained when solving the two-variable exclusive-or machine learning problem with a neural network optimized by this invention. The figure illustrates that the gained output can effectively approach the desired output when optimizing the weights of a neural network exclusive-or problem with this invention's algorithm.

To sum up, we can draw a conclusion that the heredity algorithm of this invention can effectivelyoptimize a neural network, search optimized weights and resolve a machine learning problem.

Furthermore, the network structure shown in Fig. 7 can also be encoded into gene cluster, to be optimized along with weights.

If the network structure demanded in the problem is more complex, the Heredity Programming can be implemented, so that the network structure can be optimized more directly and freely.

**Application example 2-signal processing problem**

Particle filter algorithm is an important technology in nonlinear signal processing. It is beyond the constraint of system model characteristics and noise distribution. So, it is more applicable than other filter technologies. However, the performance of particle filter algorithm is limited by particle impoverishment. This invention's algorithm is used for resolving the particle deficiency during the resampling of particle filter algorithm, optimizing the particle distribution, making the particle sample more approximate to the real posterior probability density sample, and improving the filter performance.

The status estimate of a nonlinear dynamic system is realized here through particle filter to illustrate the situation of optimized signal processing through particle filter with Heredity Algorithm, which is of great significance for finding a nonlinear filter algorithm with excellent performance. The state space model of the system is as follows:

$$\mathbf{x}_{k+1} = 1 + \sin(0.04\pi k) + 0.5\mathbf{x}_k + \mathbf{v}_k$$

$$\mathbf{z}_k = \begin{cases} 0.2\mathbf{x}_k^2 + \mathbf{n}_k & k \le 30 \\ 0.5\mathbf{x}_k - 2 + \mathbf{n}_k & k > 30 \end{cases}$$

In which, the process noise is $\mathbf{v}_k \sim Gamma(3,2)$, and the observation noise is $\mathbf{n}_k \sim N(0,0.00001)$. The observation time is set as 70, with the operation times set as 200 and the particle quantity N is set as 10.

This invention's technical solution of global optimization, search, and machine learning is applied here to resolve the particle impoverishment problem of Particle Filter algorithm and to optimize the particle distribution. A specific implementation is as follows:

Step 1: In accordance with the state estimation problem in the nonlinear dynamic system, construct an objective function $f(\mathbf{x})$. And the particle weight function is selected here;

Step 2: In accordance with the optimizing demands of the problem, automatically calculate or manually input the operation parameters of generic genetic algorithm and then initialize it.

(1) First, in accordance with the operation mode of generic genetic algorithm, the population size is determined as $S=W=10$, $N$ represents the number of particles. The variable dimensionality is set as $d=1$ with the crossover inheritance probability set as $p_c=0.9$, and the mutation probability set as $p_m=0.05$.

(2) Then, encode the structure and parameters needing optimization of the problem, form the individual gene cluster, chromosome and candidate population to determine the gene cluster's length $L$. Here, we adopt the floating number with fixed effective digits $l_x = 7$ to encode each importance sampling particle value, i.e. the status value of the system x. Each chromosome represents the floating number format of a particle. The first digit of the floating value represents the sign digit where "1" represents positive number, and "0" represents negative number. And the gene cluster's length is the fixed number of effective digits, i.e. $L = d \times l_x = 1 \times 7$. For example, when the $i$th particle state value at time is $x=10.4711$, its float-point encoding format is $\mathbf{x}_c=(1104711)$

(3) Lastly, initialize in accordance with the value range of variables in the problem, randomly produce a groups of initial candidate solutions. The initial group here is produced randomly in accordance with the particle's initial step. When $k=0$, each chromosome $\mathbf{P}_0^i$ is the coding format of a particle state value $\mathbf{x}_c=\{x_0^1(i), x_0^2(i),...,x_0^d(i)\}$, $\mathbf{P}_0^i=\{x_0^j(i), j=1,...,d\}$, $x_0^j(i)$ is each digit of the code, and the initial population is $\mathbf{G}_0=\{\mathbf{P}_0^i, i=1,2,...,S\}$.

Step 3: In line with the optimization demand, we assume that the current generation (the $k$th generation) is $G_k=\{\mathbf{P}_k^1,\mathbf{P}_k^2,...,\mathbf{P}_k^S\}$, in which, $P_k^i$ represents one individual (chromosome) in the population. The $(k+1)th$ generation, $\mathbf{G}_{k+1}=\{\mathbf{P}_{k+1}^1,\mathbf{P}_{k+1}^2,...\mathbf{P}_{k+1}^S\}$ can be gained with iterative method through overwriting and mutation operation. The optimization process is as follows:

(1) Individual evaluation: In accordance with the particle weight formula, calculate the fitness of each individual $\mathbf{P}_k^i$ in the population $\mathbf{G}_k$ after being decoded into x, which means a maximum problem, such as $f(\mathbf{P}_k^{i_1})=0.5779$ and $f(\mathbf{P}_k^{i_2})=0.4221$.

(2a) In accordance with heredity probability $p_c$, randomly select two parent chromosomes (i.e. gene clusters of parent candidate solution), such as $\mathbf{P}_k^{i_1} = \{x_k^1(i_1),x_k^2(i_1),\mathrm{K}\ x_k^j(i_1)\}_{j=1}^d$ and $\mathbf{P}_k^{i_2} = \{x_k^1(i_2),x_k^2(i_2),\mathrm{K}\ x_k^j(i_2)\}_{j=1}^d$.

(2b) Compare fitness function values of the two chromosomes $f(\mathbf{P}_k^{i_1})$ and $f(\mathbf{P}_k^{i_2})$, if $f(\mathbf{P}_k^{i_1})>f(\mathbf{P}_k^{i_2})$, $\mathbf{P}_k^{i_1}$ will pass more genes onto the offspring. Calculate genetic inheritance percentage $p_t$:

$$p_t = \frac{f(P_k^{i_1})}{f(P_k^{i_1})+f(P_k^{i_2})} = \frac{0.5779}{0.5779+0.4221} \approx \frac{3}{5}$$

Then, the number of genes passing onto the next generation $n_t$ according to the following formula is $n_t=L\cdot p_t=7\times3/5\approx5$ (rounded up to an integer)

(2c) Run heredity operator and executing overwriting operation: reserve strongly adaptable chromosome, overwrite $n_t$ genes on the strongly adaptable chromosome on corresponding position of weakly adaptable chromosome correspondingly and form the new chromosome. Corresponding overwriting is to overwrite genes on strongly adaptable chromosome on weakly adaptable chromosome. By that analogy, the basic procedure is shown in Fig. 2. In accordance with the last step, there are $7\times3/5\approx5$ code bits in $\mathbf{P}_k^{i_2}$ will be overwritten by encoding bits in $\mathbf{P}_k^{i_1}$ .

(2d) Repeat the steps from (2a)-to (2c) for $p_c$ $S$ times, produce a temporary population $\mathbf{G'}_{k+1}$ of new candidate solution after rewriting operations.

(3) Use a proper "mutation operator", execute mutation operation on temporary population $\mathbf{G'}_{k+1}$, gain the population $\mathbf{G}_{k+1}$ of the new candidate solution; since problem of machine learning nonlinear filtering is relatively complex, it is considered to adopt operation of "Use and disuse" in "mutation operator".

(3a) Generally, the non-directional uniform mutation method in common genetic algorithm can be adopted. Because mutation probability $p_m$ =0.05, $10 \times 7 \times 0.05 \approx 4$ (rounded up to an integer) code bits in new population $\mathbf{G}_{k+1}$ after one iteration will be varied, wherein if the variation bit randomly selected is sign bit of coding, it will be varied according to binary system of generic genetic algorithm.

(3b) To improve the speed and accuracy of filter algorithms, it is illustrated here with an example how to utilize the directed "Use and Disuse" operator of Heredity Algorithm to realize the non-directed "mutation operator" of generic genetic algorithm. In this application example, the "Use and Disuse" operation is realized by Hill Climbing algorithm with non-gradient optimization. Similar with the uniform mutation method, the mutation probability of the individual $\mathbf{P}_k^i$ in the new population $\mathbf{G}_{k+1}$ is also set as $p_m$ =0.05. However, it differs from non-directed mutation that we decode the individual $\mathbf{P}_k^i$ in the new population $\mathbf{G}_{k+1}$ into corresponding particle status value $x_k^i$ and then operate, which means that there will be $10\times0.05 \approx 1$ (rounded up to an integer) particle status in total involved in mutation after one iteration. The particle status after mutation will gain a new random value near the original particle status value. Moreover, the directed mutation is to obtain the best result after $N_m$ times of mutation for the particle status value $x_k^i$ to be mutated, when $N_m=S\times20\%=10\times20\%=2$ After comparing the 2 new particle state values after 2 mutations against the original $x_k^i$ when, we select the best result, the directed mutation will be completed. So, mutation always goes towards a 1 no worse direction and realizes the operation of *"Use and disuse"*.

(4) Repeat the iteration from steps (1)-(3) in the steps 3 above until the preset termination condition is met.

(5) Evaluate whether the optimal solution meets the requirements of this optimization calculation. If so, end the operation. If not, modify the algorithm's operation parameters, increase population size and iterations times, and recalculate to gain the final optimized result.

Step 4: Output the last generation's optimal solution of problem, i.e. the particle set.

Table 6 shows the comparing result of root-mean-square error (RMSE) mean value and RMSE variance after 200 times of Monte Carlo Test on condition of particle quantity being $N$=10, through the statistics of this invention and other particle filter algorithms, including basic particle filter algorithm (PF), auxiliary particle filter algorithm (APF) and regularized particle filter algorithm (RPF).,

Table 6: Test results of particle filter ($N$=10)

| Algorithm | Mean value of RMSE | Variance of RMSE |
|---|---|---|
| PF | 1.0790 | 0.0165 |
| APF | 0.8090 | 0.0134 |
| RPF | 0.9521 | 0.0118 |
| The invention | 0.4059 | 0.0043 |

Fig. 11 shows diagram of variation in RMSE mean values produced by this invention and other particle filter algorithms over time. It is illustrated from table 6 and fig. 11 that the Heredity Algorithm of this invention can better optimize particle filter, search for optimal particle list and resolve nonlinear filtering problem.

## Claims

1. A global optimization, search and machine learning method based on the Lamarckian principle of inheritance of acquired characteristics, comprising:

Step 1, constructing an objective function $f(\mathbf{P})$ according to the problem being solved for optimization, search or machine learning, where $\mathbf{P}$ represents a population set of S candidate solutions to the problem;

Step 2, encoding the population set into a genetic algorithm (GA) chromosome set comprising L genes corresponding to the number of variables of the problem and its optimization, search or machine learning needs, where each chromosome represents an individual of the population. Then, inputting or automatically calculating algorithmic parameters of the GA, and initializing the algorithm and the population at the 0*th* generation;

Step 3, supposing that the prevailing population set of candidate solutions at the *k*th generation is $\mathbf{G}_k=\{\mathbf{P}_k^1, \mathbf{P}_k^2, ..., \mathbf{P}_k^S\}$, where $P_k^i$ indicates the *i*th individual chromosome in the *k*th generation $\mathbf{G}_k$, and Sis the population size, obtaining through iteration the population for the (*k*+1)*th* generation $\mathbf{G}_{k+1}=\{\mathbf{P}_{k+1}^1, \mathbf{P}_{k+1}^2, ..., \mathbf{P}_{k+1}^S\}$, where the optimization process comprises the following steps:

(1) individual evaluation: calculating the corresponding value of objective function $f(\mathbf{P})$ for every $\mathbf{P}_k^i$ after decoding its chromosome;

(2) heredity operation: applying Lamarckian *"Heredity Operator"* to generate a temporary population $\mathbf{G}_{k+1}$, specifically including the sub-steps of:

(2a) selecting randomly two chromosomes from the candidate population Gk according to a crossover inheritance probability $p_c$ initialized in step 2, comparing objective function values $f_m$ and $f_n$ of the two chromosomes, and calculating a genetic heredity proportion $p_t$:

$$p_t=f_m/(f_m+f_n), f_m>f_n;$$

(2b) calculating the number of genes to be passed onto the next generation:

$$n_t=L\bullet p_t;$$

(2c) while retaining chromosome *m,* overwriting $n_t$ genes at random positions onto chromosome *n* at corresponding positions to form a new chromosome;

(2d) repeating steps (2a)-(2c) for $p_cS$ times to generate the temporary population $\mathbf{G'}_{k+1}$;

(3) exercising a directed mutation operation for the temporary population $\mathbf{G'}_{k+1}$ with Lamarckian *"Use-αnd Disuse Operator"* and hence forming the (*k*+1)*th* generation of candidate solutions $\mathbf{G}_{k+1}$;

(4) iterating steps (1)-(3) until the termination condition pre-specified at initialization is met;

(5) evaluating whether the best solutions in the terminating generation meet the requirements of this optimization, search or machine learning problem; if not, revising the algorithmic parameters and repeating steps (1)-(4) again until the final requirements are met;

Step 4, outputting the final solutions set and terminating the algorithm.

2. The global optimization, search and machine learning method based on the Lamarckian principle of inheritance of acquired characteristics according to claim 1, wherein in the step 2, the initializing method comprises following steps:

(1) First, confirming the algorithmic parameters according to an operation mode of a genetic algorithm, including the candidate solution population size *S*, the variable dimensionality *d*, the ranges of possible values of the variables, the number of genes in a chromosome *L,* the crossover inheritance probability $p_c$, and internal parameters in the mutation operator;

(2) Then, encoding the solution set of the problem to form individual genes, chromosomes and the candidate solution population, with length *L* of the chromosome;

(3) Last, initializing the algorithm according to the ranges of the variable values of the problem and generating one group of initial candidate solutions randomly, wherein when *k=0,* a gene group of each chromosome $\mathbf{P}_0^i$ is $\{x_0^1(i), x_0^2(i), ..., x_0^s(i)\}$, namely $\mathbf{P}_0^i=\{x_0^i(i), j=1,...,d\}$; the initial population is $\mathbf{G}_0=\{\mathbf{P}_0^i, i=1,2,...,S\}$; *S* is the population size, and *d* is the variable dimensionality.

3. The global optimization, search and machine learning method based on the Lamarckian principle of inheritance of acquired characteristics according to claim 1, wherein in the step 3 (1), the individual evaluation is implemented with multiple processors or multiple computers.

4. The global optimization, search and machine learning method based on the Lamarckian principle of inheritance of acquired characteristics according to claim 1, wherein in the step 3 (2), $n_t$ genes for overwriting are randomly selected during the overwriting operation of the Lamarckian heredity operator.

5. The global optimization, search and machine learning method based on the Lamarckian principle of inheritance of acquired characteristics according to claim 1, wherein in the step 3 (3), the mutation operation is carried out by non-directed mutation method in a generic genetic algorithm, including the uniform mutation method with mutation probability $p_m$, which don't utilize the *"Use-and-disuse operator"*.

6. The global optimization, search and machine learning method based on the Lamarckian principle of inheritance of acquired characteristics according to claim 1, wherein in the step 3 (3), the mutation operation can also be carried out by the "Use and disuse operator" based on the natural law - the *"Use-and-disuse"* of Lamarckian evolution, namely the directed mutation method.

7. The global optimization, search and machine learning method based on the Lamarckian principle of inheritance of acquired characteristics according to claim 6, wherein in the step 3 (3), gradient optimization methods can be applied to the "Use-and-disuse operator" if the gradient information is acquired; and mutation directions and step lengths can be confirmed according to the sign and size of the gradient on the premise that the gradient information is acquired.

8. The global optimization, search and machine learning method based on the Lamarckian principle of inheritance of acquired characteristics according to claim 6, wherein in the step 3 (3), non-gradient optimization methods can be applied to the "Use-and-disuse operator", which including the Hill-climbing algorithm, the Annealing algorithm, the Simplex method, the Pattern Search or the Powell's method and so on.

9. The global optimization, search and machine learning method based on the Lamarckian principle of inheritance of acquired characteristics according to claim 1, wherein in the step 3 (2) and step 3 (3), an elite chromosome in each generation shall not be changed by the Lamarckian heredity operator and the mutation operator, namely retain the elite without overwriting the best individual (also called the elite individual, or the elite chromosome) of the mutation population so far occurring due to participation in evolution; however, the best individual shall be copied directly in the next generation. The methods of confirming the elite are the methods of the generic algorithm.

10. The global optimization, search and machine learning method based on the Lamarckian principle of inheritance of acquired characteristics according to claim 1, wherein in the step 3 (5), the algorithm also includes steps: elevating an optimal solution whether meets the requirements of this optimal computation; if so, the operation ends; otherwise, crossover inheritance probability $p_c$ in algorithmic parameters or internal parameters in the mutation operator can be modified and repeating the step 3 to acquire the optimal solution set of the final generation of the problem.

11. The global optimization, search and machine learning method based on the Lamarckian principle of inheritance of acquired characteristics according to claim 1, wherein in step 3 (5), when the optimal solution does not meet the requirements of this optimization, the population size and/or iteration times in algorithmic parameters is required to be increased.

12. The global optimization, search and machine learning method based on the Lamarckian principle of inheritance of acquired characteristics according to claim 1, wherein the crossover inheritance probability $p_c$ in algorithmic parameters and internal parameters in the mutation operator, which are confirmed in the step 2, can be adjusted automatically according to the evolutional status during the step 3.

13. The global optimization, search and machine learning method based on the Lamarckian principle of inheritance of acquired characteristics according to claim 1 or 2, wherein in step 2, a gene code on the chromosome can indicate the solution structure of the problem or numerical parameters of the structure.

14. The global optimization, search and machine learning method based on the Lamarckian principle of inheritance of acquired characteristics according to claim 13, wherein in the step 2 (2), the solution set of the problem is encoded with methods: using binary or decimal encoding if $d$ is less than or equal to 2, or using real number encoding method if $d$ is greater than 2.

15. The global optimization, search and machine learning method based on the Lamarckian principle of inheritance of acquired characteristics according to claim 13, wherein in step 2, the solution set of the problem is encoded with methods: using binary or decimal encoding if d is less than or equal to 2, or using real number encoding if d is greater than 2.

16. The global optimization, search and machine learning method based on the Lamarckian principle of inheritance of acquired characteristics according to claim 15, wherein in step 2, the gene code of the solution set of the problem can also be arithmetic operators or logical operators.

Start

Step 1: Construct an objective function in accordance with a problem.

Step 2: Encode the problem into a chromosome, calculate or input operation parameters of algorithm, and initialize the algorithm.

Step 3(1): Evaluate the objective function.

Step 3(2): Run a heredity operator.

Step 3(3): Run a "Use and Disuse" operator.

Revise the algorithmic parameters

Step 3(4): Whether the predetermined ending conditions are satisfied?

No

Yes

Step 3(5): Whether the population after decoding meets the optimization requirements?

No

Yes

Step 4: Output the optimal solution set of the problem.

FIG. 1

FIG. 2

Current population $k$

New population after overwriting operation $k+1$

$\mathbf{P}_k^{i_1}$ | $x_k^1(i_1)$ | $x_k^2(i_1)$ | $x_k^3(i_1)$ | $x_k^4(i_1)$ | $x_k^5(i_1)$ | $x_k^6(i_1)$

After overwriting

New chromosome $\mathbf{P}_{k+1}^{i_1}$ =chromosome $\mathbf{P}_k^{i_1}$

$n_t$ Random Positions

0   1   1   1   0   1

New chromosome $\mathbf{P}_{k+1}^{i_2}$ =chromosome $\tilde{\mathbf{P}}_{k+1}^{i_2}$

$\mathbf{P}_k^{i_2}$ | $x_k^1(i_2)$ | $x_k^2(i_2)$ | $x_k^3(i_2)$ | $x_k^4(i_2)$ | $x_k^5(i_2)$ | $x_k^6(i_2)$

After overwriting

$x_k^1(i_2)$ | $x_k^2(i_1)$ | $x_k^3(i_1)$ | $x_k^4(i_1)$ | $x_k^5(i_2)$ | $x_k^6(i_1)$

FIG. 3

$\sqrt{h_1} + (u - 1.0)$

$h_2^3 + 1.0$

$h_2^3 + (u - 1.0)$

**Chromosome A**

**Chromosome B**

**Offspring Chromosome B***

FIG. 4

16

FIG. 5

FIG. 6

FIG. 7

FIG. 8

FIG. 9

FIG. 10

FIG. 11

## INTERNATIONAL SEARCH REPORT

| | |
|---|---|
| International application No. | |
| | PCT/CN2017/089285 |

### A. CLASSIFICATION OF SUBJECT MATTER

G06N 3/12 (2006.01) i

According to International Patent Classification (IPC) or to both national classification and IPC

### B. FIELDS SEARCHED

Minimum documentation searched (classification system followed by classification symbols)

G06N

Documentation searched other than minimum documentation to the extent that such documents are included in the fields searched

Electronic data base consulted during the international search (name of data base and, where practicable, search terms used)

WPI, EPODOC, CNKI, CNPAT: 拉马克, 遗传, 优化, 目标函数, 染色体, 交叉遗传, 种群, 基因, optimiz+, chromosome, crossover, species, gene, genetic

### C. DOCUMENTS CONSIDERED TO BE RELEVANT

| Category* | Citation of document, with indication, where appropriate, of the relevant passages | Relevant to claim No. |
|---|---|---|
| A | CN 103279796 A (SOOCHOW UNIVERSITY) 04 September 2013 (04.09.2013), description, paragraphs [0009]-[0020] | 1-16 |
| A | CN 101630380 A (XIDIAN UNIVERSITY) 20 January 2010 (20.01.2010), entire document | 1-16 |
| A | CN 106326988 A (JINGDEZHEN CERAMIC INSTITUTE) 11 January 2017 (11.01.2017), entire document | 1-16 |
| A | US 2004044633 A1 (CHEN, THOMAS W.) 04 March 2004 (04.03.2004), entire document | 1-16 |

☐ Further documents are listed in the continuation of Box C.　　☒ See patent family annex.

| | |
|---|---|
| *　　Special categories of cited documents: | "T"　later document published after the international filing date or priority date and not in conflict with the application but cited to understand the principle or theory underlying the invention |
| "A"　document defining the general state of the art which is not considered to be of particular relevance | |
| "E"　earlier application or patent but published on or after the international filing date | "X"　document of particular relevance; the claimed invention cannot be considered novel or cannot be considered to involve an inventive step when the document is taken alone |
| "L"　document which may throw doubts on priority claim(s) or which is cited to establish the publication date of another citation or other special reason (as specified) | "Y"　document of particular relevance; the claimed invention cannot be considered to involve an inventive step when the document is combined with one or more other such documents, such combination being obvious to a person skilled in the art |
| "O"　document referring to an oral disclosure, use, exhibition or other means | |
| "P"　document published prior to the international filing date but later than the priority date claimed | "&"document member of the same patent family |

| Date of the actual completion of the international search | Date of mailing of the international search report |
|---|---|
| 23 November 2017 | 20 December 2017 |

| Name and mailing address of the ISA State Intellectual Property Office of the P. R. China No. 6, Xitucheng Road, Jimenqiao Haidian District, Beijing 100088, China Facsimile No. (86-10) 62019451 | Authorized officer LI, Jia Telephone No. (86-10) 53311134 |
|---|---|

Form PCT/ISA /210 (second sheet) (July 2009)

## INTERNATIONAL SEARCH REPORT
Information on patent family members

| | International application No. |
|---|---|
| | PCT/CN2017/089285 |

| Patent Documents referred in the Report | Publication Date | Patent Family | Publication Date |
|---|---|---|---|
| CN 103279796 A | 04 September 2013 | None | |
| CN 101630380 A | 20 January 2010 | None | |
| CN 106326988 A | 11 January 2017 | None | |
| US 2004044633 A1 | 04 March 2004 | None | |

Form PCT/ISA /210 (patent family annex) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **XIN YAO.** Evolutionary Programming Made Faster. *IEEE Trans Evolutionary Computation,* 1999, vol. 3 (2), 82-101

- **MICHALEWICZ, Z.** Genetic Algorithms + Data Structure = Evolutionary Programs. Springer-Verlag, 1992